(19) Europäisches Patentamt
European Patent Office
Office européen des brevets

(11) **EP 3 545 920 B1**

(12) **EUROPÄISCHE PATENTSCHRIFT**

(45) Veröffentlichungstag und Bekanntmachung des Hinweises auf die Patenterteilung:
**06.01.2021  Patentblatt 2021/01**

(51) Int Cl.:
*A61F 9/01* (2006.01)  *A61F 9/008* (2006.01)
*A61F 9/009* (2006.01)

(21) Anmeldenummer: **19175592.5**

(22) Anmeldetag: **23.07.2004**

(54) **VORRICHTUNG ZUM AUSBILDEN GEKRÜMMTER SCHNITTFLÄCHEN IN EINEM TRANSPARENTEN MATERIAL**

DEVICE FOR FORMING BENT CROSS-SECTIONS IN A TRANSPARENT MATERIAL

DISPOSITIF DE FORMATION DE SURFACES DE COUPE COURBÉES DANS UN MATÉRIAU TRANSPARENT

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.07.2003   DE 10334110**

(43) Veröffentlichungstag der Anmeldung:
**02.10.2019   Patentblatt 2019/40**

(62) Dokumentnummer(n) der früheren Anmeldung(en) nach Art. 76 EPÜ:
**10182173.4 / 2 260 805**
**09172932.7 / 2 138 138**
**04763451.4 / 1 648 360**

(73) Patentinhaber: **Carl Zeiss Meditec AG**
**07745 Jena (DE)**

(72) Erfinder:
• **MÜHLHOFF, Dirk**
  **07751 Jena (DE)**
• **GERLACH, Mario**
  **16548 Glienicke-Nordbahn (DE)**
• **STICKER, Markus**
  **07749 Jena (DE)**
• **LANG, Carsten**
  **07607 Eisenberg (DE)**
• **BISCHOFF, Mark**
  **07749 Jena (DE)**
• **BERGT, Michael**
  **99425 Weimar (DE)**

(74) Vertreter: **Patentanwälte Geyer, Fehners & Partner mbB**
**Perhamerstrasse 31**
**80687 München (DE)**

(56) Entgegenhaltungen:
**US-A- 4 721 379     US-A- 5 993 438**
**US-A- 6 110 166**

EP 3 545 920 B1

## Beschreibung

Die Erfindung bezieht sich auf eine Vorrichtung zum Ausbilden gekrümmter Schnittflächen in einem transparenten Material, insbesondere in der Augenhornhaut, mit einer Laserstrahlungsquelle, die Laserstrahlung in das Material fokussiert und dort optische Durchbrüche bewirkt, wobei eine Scaneinrichtung, die den Fokuspunkt dreidimensional verstellt, und eine Steuereinrichtung vorgesehen sind, die die Scaneinrichtung ansteuert, um die Schnittfläche durch Aneinanderreihen der optischen Durchbrüche im Material zu bilden, und wobei die Scaneinrichtung zur Verstellung des Fokuspunktes in einer Raumrichtung eine verstellbare Optik aufweist.

Gekrümmte Schnittflächen innerhalb eines transparenten Materials werden insbesondere bei laserchirurgischen Verfahren und dort insbesondere bei augenchirurgischen Eingriffen erzeugt. Dabei wird Behandlungs-Laserstrahlung innerhalb des Gewebes d. h. unterhalb der Gewebeoberfläche derart fokussiert, dass optische Durchbrüche im Gewebe entstehen.

Im Gewebe laufen dabei zeitlich hintereinander mehrere Prozesse ab, die durch die Laserstrahlung initiiert werden. Überschreitet die Leistungsdichte der Strahlung einen Schwellwert, kommt es zu einem optischen Durchbruch, der im Material eine Plasmablase erzeugt. Diese Plasmablase wächst nach Entstehen des optischen Durchbruches durch sich ausdehnende Gase. Wird der optische Durchbruch nicht aufrechterhalten, so wird das in der Plasmablase erzeugte Gas vom umliegenden Material aufgenommen, und die Blase verschwindet wieder. Dieser Vorgang dauert allerdings sehr viel länger, als die Entstehung der Blase selbst. Wird ein Plasma an einer Materialgrenzfläche erzeugt, die durchaus auch innerhalb einer Materialstruktur liegen kann, so erfolgt ein Materialabtrag von der Grenzfläche. Man spricht dann von Photoablation. Bei einer Plasmablase, die vorher verbundene Materialschichten trennt, ist üblicherweise von Photodisruption die Rede. Der Einfachheit halber werden all solche Prozesse hier unter dem Begriff optischer Durchbruch zusammengefasst, d. h. dieser Begriff schließt nicht nur den eigentlichen optischen Durchbruch sondern auch die daraus resultierenden Wirkungen im Material ein.

Für eine hohe Genauigkeit eines laserchirurgischen Verfahrens ist es unumgänglich, eine hohe Lokalisierung der Wirkung der Laserstrahlen zu gewährleisten und Kollateralschäden in benachbartem Gewebe möglichst zu vermeiden. Es ist deshalb im Stand der Technik üblich, die Laserstrahlung gepulst anzuwenden, so dass der zur Auslösung eines optischen Durchbruchs nötige Schwellwert für die Leistungsdichte nur in den einzelnen Pulsen überschritten wird. Die US 5.984.916 zeigt diesbezüglich deutlich, dass der räumliche Bereich des optischen Durchbruchs (in diesem Fall der erzeugten Wechselwirkung) stark von der Pulsdauer abhängt. Eine hohe Fokussierung des Laserstrahls in Kombination mit sehr kurzen Pulsen erlaubt es damit, den optischen Durchbruch sehr punktgenau in einem Material einzusetzen.

Der Einsatz von gepulster Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, dass die Brechungseigenschaften von Hornhaut und Linse keine optimale Fokussierung auf der Netzhaut bewirken.

Die erwähnte US 5.984.916 sowie die US 6.110.166 beschreiben gattungsgemäße Verfahren zur Schnitterzeugung mittels geeigneter Erzeugung optischer Durchbrüche, so dass im Endeffekt die Brechungseigenschaften der Hornhaut gezielt beeinflusst werden. Eine Vielzahl von optischen Durchbrüchen wird so aneinandergesetzt, dass innerhalb der Hornhaut des Auges ein linsenförmiges Teilvolumen isoliert wird. Das vom übrigen Hornhautgewebe getrennte linsenförmige Teilvolumen wird dann über einen seitlich öffnenden Schnitt aus der Hornhaut herausgenommen. Die Gestalt des Teilvolumens ist so gewählt, dass nach Entnahme die Form und damit die Brechungseigenschaften der Hornhaut so geändert sind, dass die erwünschte Fehlsichtigkeitskorrektur bewirkt ist. Die dabei geforderten Schnittflächen sind gekrümmt, was eine dreidimensionale Verstellung des Fokus nötig macht. Es wird deshalb eine zweidimensionale Ablenkung der Laserstrahlung mit gleichzeitiger Fokusverstellung in einer dritten Raumrichtung kombiniert.

Die zweidimensionale Ablenkung der Laserstrahlung ist wie die Fokusverstellung gleichermaßen für die Genauigkeit, mit der die Schnittfläche erzeugt werden kann, ausschlaggebend. Gleichzeitig wirkt sich die Verstellgeschwindigkeit, die dabei erreichbar ist, auf die Schnelligkeit, mit der die geforderte Schnittfläche erzeugt werden kann, aus. Eine schnelle Schnittflächenerzeugung ist nicht nur aus Komfort- oder Zeitersparniswünschen anzustreben, vor dem Hintergrund, dass bei ophthalmologischen Operationen unvermeidlicherweise Bewegungen des Auges auftreten, fördert eine schnelle Schnittflächenerzeugung zusätzlich die optische Qualität des erzielten Resultats bzw. senkt die Anforderungen an eventuelle Nachführungen von Augenbewegungen.

Der Erfindung liegt deshalb die Aufgabe zugrunde eine Vorrichtung der eingangs genannten Art so auszugestaiten, dass für die Erzeugung einer Schnittfläche eine möglichst geringe Zeit erforderlich ist.

Die Aufgabe wird mit einer Vorrichtung gemäß Hauptanspruch 1 gelöst, bei der die Steuereinrichtung die Scaneinrichtung so ansteuert, dass der Fokuspunkt in den übrigen zwei Raumrichtungen auf Höhenlinien der Schnittfläche geführt ist, welche in Ebenen liegen, die senkrecht zur ersten Raumrichtung sind. Bevorzugte Ausführungsformen der Erfindung werden in den abhängigen Ansprüchen aufgeführt.

Erfindungsgemäß werden also bei der Erzeugung der optischen Durchbrüche Bahnen verwendet, denen Höhenlinien der zu erzeugenden Schnittfläche zugrunde liegen. Die Höhenlinien sind dabei auf diejenige Raumrichtung des Systems bezogen, in der die langsamste Verstellgeschwindigkeit gegeben ist. Es ist dadurch möglich, den Fokus in dieser Raumrichtung über einen längeren Zeitraum nahezu unverändert zu lassen, und die höhere Verstellgeschwindigkeit in den anderen beiden Raumrichtungen kann ohne Begrenzung ausgeschöpft werden. Man erhält insgesamt eine schnelle Schnittflächenerzeugung. Die Höhenlinien kann man zweckmäßigerweise durch Schneiden der gekrümmten Schnittfläche mit Ebene senkrecht zur ersten Raumrichtung erhalten. Je exakter die Ebenen der Höhenlinien senkrecht zur ersten Raumrichtung stehen, desto konstanter kann die Verstellung in der ersten Raumrichtung während einer Höhenlinie gehalten werden.

Die Laserstrahlung wird dazu zumindest bezogen auf die zwei Raumrichtungen, die senkrecht zur Ebene der Höhenlinie liegen, unter Berücksichtigung des Höhenlinienverlaufes verstellt. Dabei ist es zum einen möglich, dass der Fokuspunkt innerhalb gewisser Toleranzen exakt der jeweiligen Höhenlinie folgt. In diesem Fall beschreibt der Fokuspunkt konzentrisch gelegene geschlossene Bahnkurven, wobei für jede Bahnkurve der Fokus in der ersten Raumrichtung entsprechend unterschiedlich eingestellt ist. Anstelle von geschlossenen Bahnkurven, die den Höhenlinien innerhalb bestimmter Toleranzen exakt folgen, ist es auch möglich, die Höhenlinien zusammenhängend miteinander zu verbinden. Der Fokuspunkt wird dabei entlang einer Höhenlinie bewegt, wobei die einzelnen Höhenlinien nicht als geschlossene Bahnkurven ausgeführt werden, sondern benachbarte Höhenlinien durch einen gleitenden Übergang miteinander verbunden sind, so dass insgesamt der Fokuspunkt auf einer einzigen zusammenhängenden Bahnkurve bewegt wird. Es entsteht dadurch eine auf einer geschlossenen Bahnkurve liegende Reihe optischer Durchbrüche, die die Schnittfläche bilden. Diese ununterbrochene Aneinanderreihung von Höhenlinien kann vorzugsweise dadurch erreicht werden, dass der Fokuspunkt jeweils bis auf ein Reststück vollständig entlang der Höhenlinie bewegt wird und im Reststück durch Verstellung des Fokuspunktes in der ersten Raumrichtung ein Übergang zur nächsten Höhenlinie bewirkt wird. Dieser Ansatz hat den Vorteil, dass die Anforderungen an die Verstellung in der ersten Raumrichtung nochmals gesenkt sind, da auch während des Übergangs zwischen zwei Höhenlinien optische Durchbrüche zur Bildung der Schnittfläche erzeugt werden.

Das Höhenlinienbild wird dabei von der Topographie, d. h. der Krümmung der Schnittfläche abhängen. Bei einer sphärisch gekrümmten Schnittfläche erhält man konzentrische kreisförmige Höhenlinien. Da bei augenoptischen Korrekturen regelmäßig auch ein gewisser Astigmatismus zu korrigieren ist, wird jedoch eine sphärisch gekrümmte Schnittfläche eher die Ausnahme, eine Ellipsoid- oder Toroidfläche dagegen meist die Regel sein. Für eine solche Ellipsoidfläche sind die Höhenlinien als (günstigerweise konzentrische) Ellipsen ausgebildet. Vorzugsweise liegt die Elliptizität zwischen 1,0 und 1,1 oder sogar 1,2.

Bei einer solchen Form können die Höhenlinien auch derart der Führung des Fokuspunktes zugrunde gelegt werden, dass der abgelenkte Fokuspunkt einer Ellipsoid-Spirale folgt, d. h. einer auf der Mantelfläche der gekrümmten Schnittfläche liegenden Spirale.

Die Elliptizität der Ellipsen bzw. der Ellipsoid-Spirale kann dabei von der Form der Hornhautoberfläche abhängen. Unter Elliptizität ist das Verhältnis der großen Hauptachse einer Ellipse zu ihrer kleinen Hauptachse zu verstehen.

Für kontaktlose Verfahren wird mit der natürlichen Oberflächentopographie gearbeitet, bei Verwendung eines Kontaktglases spielt die Form dieses Kontaktglases eine Rolle. Hier besteht ein Vorteil des Ansatzes mit Verwendung eines Kontaktglases, da die Topographie mit angepresstem Kontaktglas wohl definiert ist. Ein ebenes Kontaktglas stellt einen mathematischen Grenzfall dar, und das Konzept des Höhenlinien-Scans führt hier zur Entartung der Bahnkurven, obwohl diese auch noch als geschlossen bezeichnet werden können. Für den auch aus applikativer Sicht interessanteren Fall eines gekrümmten Kontaktglases ergibt sich eine Abhängigkeit der Oberflächentopographie, z. B. der Elliptizität von der Krümmung des Kontaktglases. Dies gilt auch, wenn die Krümmung rein sphärisch ist, da sich dann ebenfalls eine Ellipsoidform für die Schnittfläche einstellt. Die Elliptizität ist allerdings meist nicht im ganzen Bearbeitungsfeld gleich, sondern zeigt oft eine radiale Abhängigkeit.

Im Prinzip ergibt sich für die Elliptizität e folgender Zusammenhang:

$$e(z) = \frac{\sqrt{R_a^2 - (R_a - z)^2}}{\sqrt{R_b^2 - (R_b - z)^2}},$$

wobei $R_a$ und $R_b$ die Krümmungsradien der Cornea-Oberfläche in Richtung der Hauptachsen der Ellipse bezeichnen und z der Abstand des Bearbeitungspunktes (der Höhenlinie) vom Hornhautscheitel ist. Da z dann eine Funktion des radialen Parameters des Bearbeitungsfeldes (Abstand zur optischen Achse) ist, ist es zweckmäßig, die bereits erwähnte radiale Abhängigkeit der Elliptizität als e(z) = e(z(r)) zu wählen.

Die oben genannte Gleichung gilt primär für das unkontaktierte Auge, da auch dort, wie oben erwähnt, meist eine Ellipsoidform vorliegt. Bei Anpressen an ein Kontaktglas kommt es in der Regel zu einer Deformation, die bei der Berechnung berücksichtigt wird. Dabei spielen neben den Kugelkoordinaten $R, \varphi, \alpha$ im natürlichen Augensystem und im Kontaktglassystem (gestrichene Koordinaten) der äußere Krümmungsradius der Cornea $R_{Cv}$ und der Krümmungsradius des Kontaktglases $R_G$ eine Rolle. Eine einfache und kompakte Form der Transformationsgleichungen für diese Anpresstransformation lautet:

$$\varphi' = \varphi$$

$$\alpha' \cdot R' = \alpha \cdot R$$

$$R_G - R' = R_{Cv} - R$$

Weitere Ergänzungen, die in den Gleichungen zu Korrekturtermen führen, sind natürlich möglich und teilweise auch sinnvoll. Der hier offenbarte heuristische Ansatz wird dadurch jedoch nur modifiziert, gilt also im Prinzip weiter. Die genannten Zusammenhänge ermöglichen eine einfache Berechnung der Bahnkurven, was auch die Berechnung der Elliptizität mit einschließt. Ein besonders wichtiger Schritt bei den Algorithmen zur Berechnung ist die oben angegebene Vorwärts- und Rückwärtstransformation zwischen natürlichem Augensystem und Kontaktglassystem .

Die Elliptizität der Bahnkurven beträgt für ein Kontaktglas mit einem Krümmungsradius, der etwa dem des menschlichen Auges entspricht, in der Regel weniger als 1.4 (große Hauptachse 10% länger als die kleine Hauptachse). Die Elliptizität beträgt bei einer sphäro-zylindrischen Korrektur mit -2dpt und 1dpt beispielsweise nur etwa 1.03 im zentralen Feldbereich nahe der optischen Achse und wächst mit dem Abstand von der optischen Achse bis zur äußeren Bahnkurve um ca. 10%. Für eine praktikable Ausführungsform spielt die Veränderlichkeit der Elliptizität oder einer entsprechenden Modifikation einer idealen Kreisbahn bei Fehlsichtigkeitskorrektur höherer Ordnung keine störende Rolle, kann also in einer ersten Näherung als konstant angenommen werden.

Die Abstände zwischen den der Steuerung zugrunde zu legenden Höhenlinien sind naturgemäß durch die Abstände der Ebenen gegeben, die durch einen mathematischen Schnitt mit der gekrümmten Schnittfläche die Höhenlinien erzeugt sind. Um sicherzustellen, dass die Vielzahl von optischen Durchbrüchen eine zusammenhängende Schnittfläche ausbildet, ist darauf zu achten, dass der Maximalabstand der Höhenlinien einen Grenzwert nicht überschreitet. Es ist deshalb zweckmäßigerweise zu bevorzugen, dass Abstände der Höhenlinien in der ersten Raumrichtung so gewählt werden, dass die Abstände zwischen benachbarten Höhenlinien einen Grenzwert nicht überschreiten. Dabei kann als zu überprüfendes Maß sowohl der Abstand im Höhenlinienbild als auch der Abstand im dreidimensionalen Raum verwendet werden. Da in der Augenchirurgie die gekrümmten Schnittflächen zur optischen Korrektur innerhalb gewisser Grenzen in oftmals ausreichender Näherung einer sphärischen bzw. einer Ellipsoid-Geometrie genügen, kann es zur Vereinfachung ausreichen, dass die Abstände in der ersten Raumrichtung so gewählt werden, dass die mittleren Abstände der Höhenlinien konstant sind und insbesondere unterhalb eines Schwellwertes liegen, der natürlich geringer ist als der vorgenannte Grenzwert. Bei ellipsoidförmigen Schnittflächen kann vereinfacht im Höhenlinienbild der Abstand benachbarter Höhenlinien an der langen Halbachse ausgewertet werden, um sicherzustellen, dass die optischen Durchbrüche ausreichend dicht liegen.

Bei ophthalmologischen Operationen kann es mitunter erforderlich werden, auch höhere Aberrationen durch Entfernen eines Volumens aus der Hornhaut zu korrigieren. Die dazu erforderliche gekrümmte Schnittfläche weist dementsprechend dann auch höhere Krümmungsordnungen auf. Will man diese Formen durch Höhenlinien direkt abbilden, ergibt sich mitunter ein sehr komplexes Höhenlinienbild, das eine komplexe und schnelle Verstellung in den zwei übrigen Raumrichtungen beim Abfahren einer Höhenlinie erfordert. Für solche Fälle ist es zweckmäßig, bei der Bestimmung der Höhenlinien die höheren Krümmungsordnungen der gekrümmten Schnittfläche zu vernachlässigen und dann, während der Fokuspunkt in den übrigen zwei Raumrichtungen gemäß der Höhenlinie verstellt wird, die Verstellung in der ersten Raumrichtung gemäß dem Einfluss der höheren Krümmungsordnungen zu verändern. Die Korrektur höherer Aberrationen wird also dann in der ersten Raumrichtung, z. B. in z-Richtung auf eine Grundbewegung aufmoduliert, die der gekrümmten Schnittfläche ohne höhere Aberrationen entspricht.

Bei vielen augenoptischen Korrekturen ist es aufgrund physiologischer Gegebenheiten vorteilhaft, zur Fehlsichtigkeitskorrektur ein Volumen zu entnehmen, das bezogen auf die optische Achse des Auges in einem kreisförmigbegrenzten Bereich liegt. Dies gilt auch, falls astigmatische Korrekturen nötig sind. Für solche Fälle ist es vorteilhaft, mittels der Höhenlinien eine Ellipse abzutasten, in den Randbereichen, in denen die Ellipse über den gewünschten kreisförmigen Bereich hinausragt, jedoch die Laserstrahlung (z. B. durch einen optischen Schalter oder eine Blende oder durch Eingriff an der Laserstrahlungsquelle) so zu steuern, dass dort keine optischen Durchbrüche bewirkt werden. Durch eine derartige Ausblendung von Randbereichen der Ellipse kann sichergestellt werden, dass die (astigmatisch) gekrümmte Schnittfläche nur in einem kreisförmigen Bereich erzeugt wird.

In der erfindungsgemäßen Vorrichtung kann die Verstellung des Fokuspunktes mit einer Scaneinrichtung bewirkt werden, die zur Verstellung in der ersten Raumrichtung (üblicherweise z-Richtung) ein vorzugsweise als abstimmbares Teleskop ausgebildetes Zoom-Objektiv und für die anderen beiden Raumrichtungen (üblicherweise x- und $\gamma$-Richtungen) zwei Kippspiegel mit gekreuzten Drehachsen aufweist.

Für die durch optische Mittel bewirkte Ausbildung gekrümmter Schnittflächen ist es vorteilhaft, wenn die Oberfläche des Materials, insbesondere die Augenhornhautvorderfläche, eine bekannte Form hat. Dies erleichtert die Führung des Fokuspunktes. Weiter ist es zweckmäßig, das zu bearbeitende Material, insbesondere die Augenhornhaut, räumlich zu fixieren, da man dann auf mitunter aufwendige Strahlnachführungen verzichten kann. Unter beiden Gesichtspunkten ist es zweckmäßig, auf das Material ein Kontaktglas aufzusetzen, das der Materialoberfläche eine bestimmte Form gibt. Diese Form wird dann bei der Bestimmung der Höhenlinien berücksichtigt. Dies kann insbesondere dadurch erfolgen, dass die eingangs erwähnte Koordinatentransformation, die durch das Anpressen an das Kontaktglas erfolgt, bei der Ansteuerung eingeht.

Die Verwendung eines Kontaktglases ist sowohl für das erfindungsgemäße Verfahren als auch für die erfindungsgemäße Vorrichtung vorteilhaft. Bei der Vorrichtung ist die vom Kontaktglas der Oberfläche des Materials verliehene Form in der Steuereinrichtung bekannt oder wird dieser geeignet eingegeben, so dass die Steuereinrichtung bei der Wahl der Höhenlinien die Oberflächenform des Materials zugrunde legt.

Die Erfindung wird nachfolgend unter Bezugnahme auf die Zeichnung beispielhalber noch näher erläutert. In der Zeichnung zeigt:

| | |
|---|---|
| Figur 1 | eine perspektivische Darstellung eines Patienten während einer laserchirurgischen Behandlung mit einem laserchirurgischen Instrument, |
| Figur 2 | die Fokussierung eines Strahlenbündels auf das Auge des Patienten beim Instrument der Figur 1, |
| Figur 3 | eine schematische Darstellung zur Erläuterung einer während der laserchirurgischen Behandlung mit dem Instrument der Figur 1 erzeugten Schnittfläche, |
| Figur 4 | eine Ablenkvorrichtung des laserchirurgischen Instruments der Figur 1, |
| Figur 5 | ein beispielhaftes Höhenlinienbild, das bei der Ansteuerung der Ablenkeinrichtung der Figur 4 zugrunde gelegt wird, |
| Figur 6 | einen Ausschnitt eines Höhenlinienbilds ähnlich dem der Figur 5 zur Verdeutlichung des Übergangs zwischen aufeinanderfolgenden Höhenlinien, |
| Figur 7 | ähnlich der Figur 6 mit einer weiteren Möglichkeit für einen Übergang zwischen Höhenlinien, |
| Figuren | 8a und 8b ein weiteres Beispiel für ein Höhenlinienbild samt zugehörigen Ansteuerfunktionen für die Ablenkvorrichtung der Figur 4, |
| Figur 9 | eine Draufsicht auf einen Schnittbereich beim Ausführen einer augenoptischen Operation zur Fehlsichtigkeitskorrektur, |
| Figur 10 | eine Darstellung ähnlich der Figur 2 bei Verwendung eines Kontaktglases, |
| Figur 11 | bei der Bestimmung der Höhenlinien relevante Größen und |
| Figuren 12 und 13 | die Größen der Figur 11 mit und ohne Kontaktglas. |

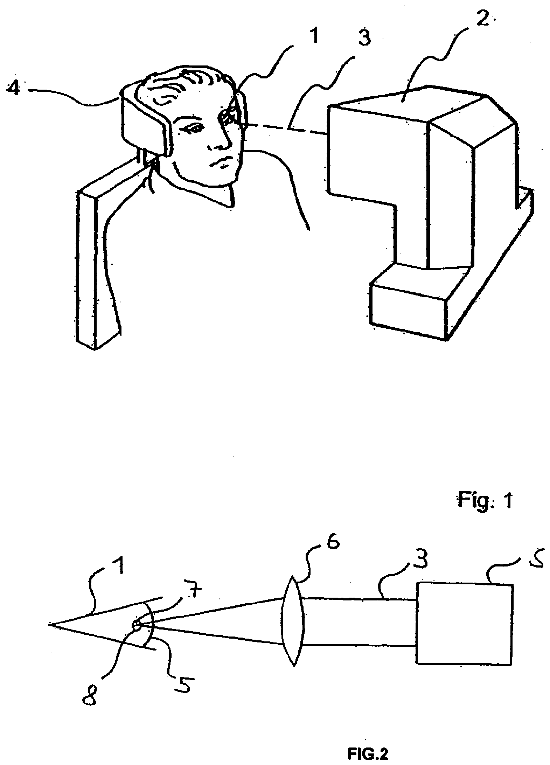

In Figur 1 ist ein laserchirurgisches Instrument zur Behandlung eines Auges 1 eines Patienten gezeigt, wobei das laserchirurgische Instrument 2 zur Ausführung einer refraktiven Korrektur dient. Das Instrument 2 gibt dazu einen Behandlungs-Laserstrahl 3 auf das Auge des Patienten 1 ab, dessen Kopf in einen Kopfhalter 4 fixiert ist. Das laserchirurgische Instrument 2 ist in der Lage, einen gepulsten Laserstrahl 3 zu erzeugen, so dass das in US 6.110.166 beschriebene Verfahren ausgeführt werden kann.

Das laserchirurgische Instrument 2 weist dazu, wie in Figur 2 schematisch dargestellt ist, eine Strahlquelle S auf, deren Strahlung in die Hornhaut 5 des Auges 1 fokussiert wird. Mittels des laserchirurgischen Instrumentes 2 wird eine Fehlsichtigkeit des Auges 1 des Patienten dadurch behoben, dass aus der Hornhaut 5 Material so entfernt wird, dass sich die Brechungseigenschaften der Hornhaut um ein gewünschtes Maß ändern. Das Material wird dabei dem Stroma der Hornhaut entnommen, das unterhalb von Epithel und Bowmanscher Membran oberhalb der Decemetschen Membran und des Endothels liegt.

Die Materialentfernung erfolgt, indem durch Fokussierung des hochenergetischen gepulsten Laserstrahls 3 mittels eines objektiven Teleskops 6 in einem in der Hornhaut 5 liegenden Fokus 7 in der Hornhaut Gewebeschichten getrennt werden. Jeder Puls der gepulsten Laserstrahlung 3 erzeugt dabei einen optischen Durchbruch im Gewebe, welcher eine Plasmablase 8 initiiert. Dadurch umfasst die Gewebeschichttrennung ein größeres Gebiet, als der Fokus 7 der Laserstrahlung 3. Durch geeignete Ablenkung des Laserstrahls 3 werden nun während der Behandlung viele Plasmablasen 8 aneinandergereiht. Die aneinanderliegenden Plasmablasen 8 bilden dann eine Schnittfläche 9, die ein Teilvolumen T des Stromas, nämlich das zu entfernende Material der Hornhaut 5 umschreiben.

Das laserchirurgische Instrument 2 wirkt durch die Laserstrahlung 3 wie ein chirurgisches Messer, das, ohne die Oberfläche der Hornhaut 5 zu verletzen, direkt Materialschichten im Inneren der Hornhaut 5 trennt. Führt man den Schnitt durch weitere Erzeugung von Plasmablasen 8 bis an die Oberfläche der Hornhaut 5, kann ein durch die Schnittfläche 9 isoliertes Material der Hornhaut 5 seitlich herausgezogen und somit entfernt werden.

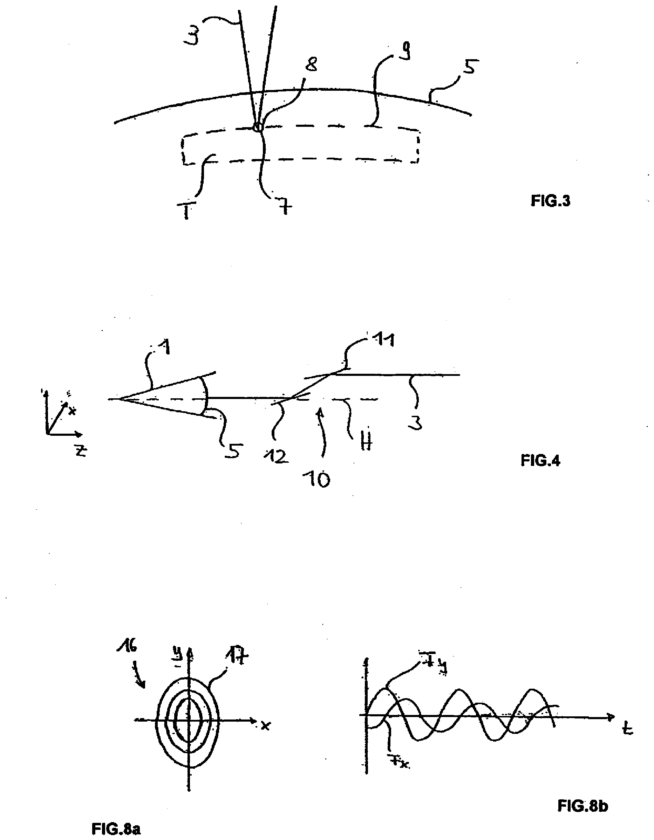

Die Erzeugung der Schnittfläche 9 mittels des laserchirurgischen Instrumentes 2 ist in Figur 3 schematisch dargestellt. Durch Aneinanderreihung der Plasmablasen 8 in Folge stetiger Verschiebung des Fokus 7 des gepulsten fokussierten Laserstrahls 3 wird die Schnittfläche 9 gebildet.

Die Fokusverschiebung erfolgt dabei zum einen in einer Ausführungsform mittels der in Figur 4 schematisch dargestellten Ablenkeinheit 10, die den auf einer Haupteinfallsachse H auf das Auge 1 einfallenden Laserstrahl 3 um zwei senkrecht zueinander liegenden Achsen ablenkt. Die Ablenkeinheit 10 verwendet dafür einen Zeilenspiegel 11 sowie einen Bildspiegel 12, was zu zwei hintereinander liegenden räumlichen Ablenkachsen führt. Der Kreuzungspunkt der Hauptstrahlachse mit der Ablenkachse ist dann der jeweilige Ablenkpunkt. Zur Fokusverschiebung wird zum anderen das Teleskop 6 geeignet verstellt. Dadurch kann der Fokus 7 in dem in Figur 4 schematisch dargestelltem x/y/z-Koordinatensystem entlang dreier orthogonaler Achsen verstellt werden. Die Ablenkeinheit 10 verstellt den Fokus in der x/y-Ebene, wobei der Zeilenspiegel den Fokus in der x-Richtung und der Bildspiegel in der $\gamma$-Richtung zu verstellen erlaubt. Das Teleskop 6 wirkt dagegen auf die z-Koordinate des Fokus 7.

Ist eine wie in Fig. 3 gezeigte Schnittfläche 9 in die gleiche Richtung wie die Hornhautoberfläche gewölbt, so ist dies mit einer Optik, deren Bildfeldkrümmung ähnlich der Krümmung der Hornhaut ist, zu erreichen, ohne dass die Führung des Fokus 7 dies berücksichtigen muss.

Aufgrund der Corneakrümmung, die zwischen 7 und 10 mm beträgt, ist das Teilvolumen T auch entsprechend gekrümmt. Die Corneakrümmung wirkt sich somit in Form einer Bildfeldkrümmung aus. Diese wird durch geeignete Ansteuerung der Ablenkeinheit berücksichtigt.

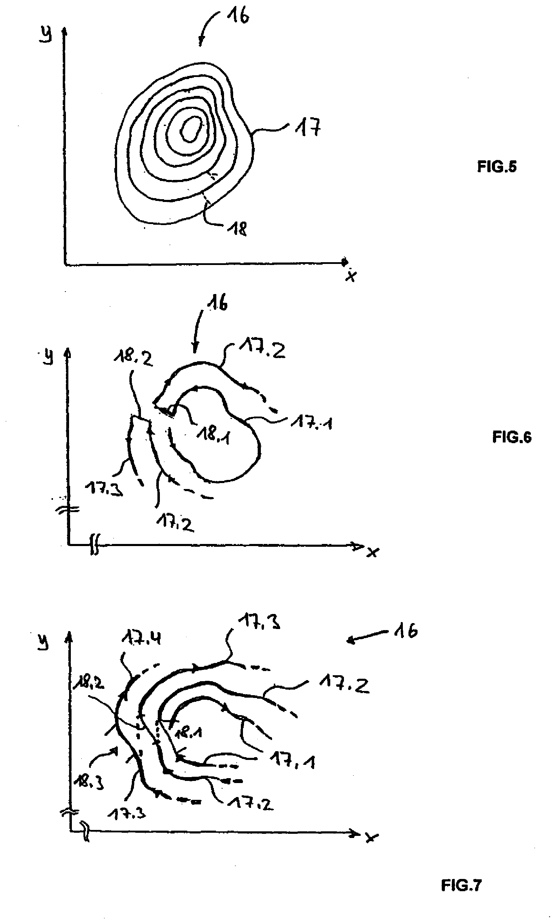

Zur Erzeugung der Schnittfläche 9 wird aus deren Krümmung ein Höhenlinienbild 16 bestimmt, wie es beispielshalber in Figur 5 in der x/y-Ebene dargestellt ist. Das Höhenlinienbild 16 besteht aus einer Vielzahl konzentrischer Höhenlinien 17, die Punkte gleicher z-Koordinaten der Schnittfläche 9 verbindet. Das Höhenlinienbild 16 wurde gewonnen, in dem aus der gekrümmten Schnittfläche 9 diejenigen Punkte bestimmt, z. B. herausgefiltert wurden, die zumindest näherungsweise eine bestimmte z-Koordinate haben. Dies entspricht einem mathematischen Schnitt der gekrümmten Schnittfläche 9 mit einer x/y-Ebene mit der jeweiligen z-Koordinate. Die z-Koordinaten wurden dabei zur Erzeugung der einzelnen Höhenlinien 17 des Höhenlinienbildes 16 der Figur 5 so gewählt, dass die Abstände benachbarter der Höhenlinien 17 im Höhenlinienbild 16 einen vorbestimmten Grenzwert nicht überschreiten. Dieser Grenzwert ist durch den maximal zulässigen Abstand zweier Plasmablasen 8 festgelegt, der zum Erreichen einer zusammenhängenden Schnittfläche zulässig ist.

Zum Erzeugen der Schnittfläche 9 wird nun der Fokus 7 entsprechend der Höhenlinien 17 durch die Ablenkeinheit 10 verstellt, wobei die Zoom-Optik 6 für jede Höhenlinie 17 die entsprechende z-Koordinate für den Fokus 7 einstellt. Während der Fokus 7 über eine Höhenlinie 17 läuft, bleibt das Teleskop 6 fest eingestellt. Lediglich während in Figur 5 gestrichelt eingezeichneten Übergängen 18 zwischen benachbarten Höhenlinien erfolgt eine Verstellung.

Figur 6 zeigt einen Ausschnitt des Höhenlinienbildes 16. Jede Höhenlinie 17 wird dabei als fast vollständig geschlossene Kurve vom Fokus 7 abgefahren, wobei der Abstand zwischen Anfang und Ende einer Höhenlinie 17 den durch den Grenzwert definierten zulässigen maximalen Abstand zwischen zwei Plasmablasen 8 nicht überschreitet. Am Ende einer jeden Höhenlinie 17 (in Figur 6 sind drei Höhenlinien 17.1, 17.2 und 17.3 angedeutet) erfolgt ein Übergang 18 durch Verstellen des Teleskopes 6 zur jeweils nächsten Höhenlinie. Zwischen den Höhenlinien 17.1 und 17.2 liegt dadurch ein Übergang 18.1, zwischen den Höhenlinien 17.2 und 17.3 ein Übergang 18.2. Dies setzt sich für alle Höhenlinien fort. Durch den derart gewählten Übergang ist zum einen erreicht, dass der Grenzwert für den maximal zulässigen Abstand zwischen zwei Plasmablasen 8 nicht überschritten wird, zum anderen die Höhenlinien 17 als zusammenhängende Spur geschrieben werden kann.

In Figur 6 liegen die Übergänge 18 im Wesentlichen auf Falllinien der gekrümmten Schnittfläche 9. Figur 7 zeigt diesbezüglich andere Übergänge 18.1 bis 18.3, bei denen ein gleitender Übergang zwischen dem Ende einer Höhenlinie und dem Beginn der unmittelbar benachbarten Höhenlinie erfolgt. Zur Verdeutlichung ist die vom Fokus 7 nicht verfolgte Fortsetzung der entsprechenden Höhenlinien in Figur 7 gestrichelt eingezeichnet. Wie zu sehen ist, wird am Ende einer Höhenlinie 17 ein gleitender Übergang auf die nächste Höhenlinie durch geeignete Ansteuerung des Zeilenspiegels 11 sowie des Bildspiegels 12 vorgenommen. Gleichzeitig wird synchron das Teleskop 6 während der dadurch erreichten Übergänge 18.1, 18.2 und 18.3 verstellt.

Es ergibt sich dadurch im Gegensatz zum Übergang der Figur 6, bei der benachbarte Höhenlinien in entgegengesetzter Umlaufrichtung durchlaufen werden, ein gleichsinniger Umlauf um die Höhenlinien, die ähnlich einer Spirale aneinandergereiht werden. Im Unterschied zu einer wirklichen Spirale wird jedoch bis auf den Übergang 18 die Höhenlinie durch den Fokus 7 abgefahren und der Wechsel von einer Höhenlinie zur nächsten erfolgt über einen kleinen Winkelbereich des Umlaufs statt kontinuierlich während eines 360° Umlaufes.

Figur 8a zeigt ein weiteres Beispiel für ein Höhenlinienbild 16, das hier aus konzentrischen elliptischen Höhenlinien 17 aufgebaut ist. Für dieses Höhenlinienbild ist für jede Höhenlinie 17 die in Figur 8b schematisch dargestellte zeitliche Ansteuerung von Zeilenspiegel 11 und Bildspiegel 12 vorgesehen, die hier mit Ansteuerfunktionen Fy und Fx angesteuert werden, die der Gleichung $\sin\varphi$ bzw. $A \cdot \sin(\varphi+\alpha)$ und $\cos\varphi$ bzw. $R \cdot \cos(\varphi+\alpha)$ genügen (mit $\varphi$ Winkelpa-

rameter der Höhenlinie, $\alpha$ auf die Winkellage der Ellipsenhauptachse zur $\gamma$-Achse wirkendem Parameter R, und A die Elliptizität beeinflussendem Parameter, wobei meist R=1 gilt).

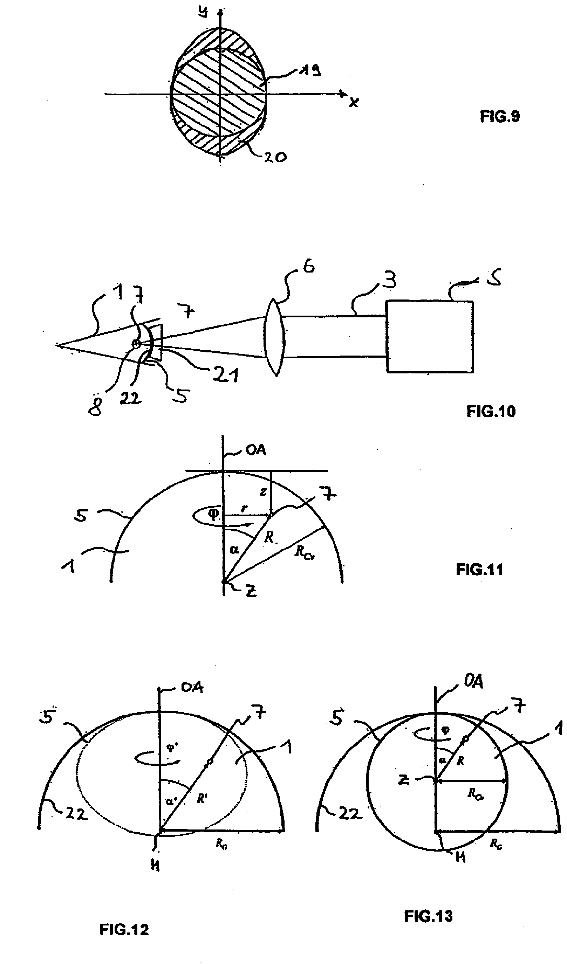

Da bei einem nichtkreisförmigen Höhenlinienbild die Schnittfläche 9, in z-Richtung gesehen einen nichtkreisförmigen Bereich umfasste, was aus ophthalmologischer Sicht nicht wünschenswert ist, wird in einer Ausführungsform bei solchen, nicht rotationssymmetrischen Höhenlinienbildern in Bereichen, die außerhalb eines kreisförmigen Bereiches liegen, die Strahlquelle S so gesteuert, dass sie im Material 5 keinen optischen Durchbruch, d. h. keine Plasmablase 8 erzeugt. Dies ist in Figur 9 durch unterschiedliche Schraffuren dargestellt. Im von links oben nach rechts unten schraffierten kreisförmigen Bereich 19 kann die Strahlquelle S Plasmablasen 8 erzeugen. In den darüber hinausragenden Bereichen 20, in denen das Höhenlinienbild 16 den gewünschten kreisförmigen Bereich 19 verlässt, ist die Strahlquelle S dagegen abgeschaltet oder wird zumindest so betrieben, dass keine Plasmablasen 8 entstehen können.

Bisher wurde das laserchirurgische Instrument 2 sowie das dadurch ausgeführte Verfahren in Zusammenhang mit einem Konzept beschrieben, das die Form der Vorderfläche der Augenhornhaut für den Eingriff unverändert lässt. Obige Beschreibung gilt jedoch auch für Ansätze, die auf die Augenhornhaut 5 ein Kontaktglas aufsetzen. Der bei einem solchen Vorgehen vorliegende Aufbau ist schematisch in Figur 10 gezeigt, die im Wesentlichen der Figur 2 entspricht, so dass auf dort bereits beschriebene Elemente nicht weiter eingegangen wird. Im Unterschied zu Figur 2 ist auf die Augenhornhaut 5 jetzt allerdings ein Kontaktglas 21 aufgesetzt, das mit seiner Innenfläche 22 der Vorderfläche der Augenhornhaut 5 ein bestimmtes Profil verleiht. Im Unterschied zum bisher beschriebenen Vorgehen, ist nun bei der Bestimmung der Bahnkurven, z. B. der Höhenlinien, nicht mehr die Krümmung der Augenhornhaut 5 im freien, d. h. natürlichen Zustand, zugrunde zu legen, sondern die durch die Innenfläche 22 des Kontaktglases 21 vorgegebene Form.

Ohne Kontaktglas 21 stellen sich die geometrischen Verhältnisse am Auge 1 wie in Figur 11 gezeigt dar. Die Augenhornhaut 5 ist bezogen auf das Augenzentrum Z näherungsweise sphärisch gekrümmt, so dass sie durch einen Krümmungsradius $R_{Cv}$ sowie die Lage des Zentrums Z auf der optischen Achse OA eindeutig lagebestimmt ist. Die Koordinaten eines Punktes, an dem ein Laserfokus 7 zu liegen kommt, um eine Plasmablase 8 zu erzeugen, sind damit entweder in Zylinderkoordinaten (Radius r von der optischen Achse OA, Abstand z von der Scheitelpunktebene und Winkel $\varphi$) oder in radialen Koordinaten (Radius r vom Augenzentrum Z, Winkel $\varphi$ und a) eindeutig angebbar. In beiden Koordinatensystemen können die Höhenlinien bzw. die Bahnkurven, an denen der Fokus 7 verstellt wird, berechnet und angegeben werden, wobei in Zylinderkoordinaten elliptische Bahnkurven besonders einfach mathematisch beschrieben werden können.

Setzt man nun auf das Auge ein Kontaktglas 21 auf, liegen die in Figur 13 gezeigten Verhältnisse vor, solange das Kontaktglas 21 mit seiner Innenfläche 22 die Augenhornhaut nicht verformt. Das Kontaktglas ist hier sphärisch gekrümmt, wobei der Krümmungsradius $R_G$ größer ist, als der Krümmungsradius $R_{Cv}$ der Augenhornhaut. Presst man nun das Kontaktglas 21 auf das Auge 1, verformt sich die Augenhornhaut 5 von einer Sphäre zu einem Ellipsoid; es stellen sich die in Figur 12 schematisch dargestellten Verhältnisse ein. Das Anpressen bewirkt also die Verformung des Auges, das sich zumindest in einem Bereich um die optische Achse OA sehr viel enger an die Innenfläche 22 des Kontaktglases 21 anlegt, als ohne Anpressung.

Da sich die geometrischen Verhältnisse nun ändern, kann man den Vorgang des Anpressens bezüglich der mathematischen Beschreibung der Orte der Fokuspunkte 7 und damit der Bahnkurven in einer Koordinatentransformation, die auch als "Anpresstransformation" bezeichnet wird, fassen. Die transformierten Koordinaten werden dann zweckmäßigerweise auf den Mittelpunkt M des vorzugsweise sphärisch gekrümmten Kontaktglases bezogen, da das Kontaktglas üblicherweise auch zur Fixierung des Auges 1 verwendet wird, d. h. fest mit dem Instrument 2 verbunden ist. Hier wirkt sich die Doppelfunktion des Kontaktglases (Formgebung und räumliche Fixierung) aus.

Es stellen sich dann elliptische Bahnkurven ein. Die Elliptizität der Bahnkurven hängt von der Form diese Kontaktglases ab. Unter Elliptizität ist das Verhältnis der großen Hauptachse einer Ellipse zu ihrer kleinen Hauptachse zu verstehen.

Ein ebenes Kontaktglas stellt einen mathematischen Grenzfall dar, und das Konzept des Höhenlinien-Scans führt hier zur Entartung der Bahnkurven, obwohl diese auch noch als geschlossen bezeichnet werden können. Für den auch aus applikativer Sicht interessanteren Fall eines gekrümmten Kontaktglases ergibt sich eine Abhängigkeit der Elliptizität von der Krümmung des Kontaktglases. Die Elliptizität ist zudem meist nicht im ganzen Bearbeitungsfeld gleich, sondern zeigt eine radiale Abhängigkeit.

Im Prinzip gilt für die Elliptizität e:

$$e(z) = \frac{\sqrt{R_a^2 - (R_a - z)^2}}{\sqrt{R_b^2 - (R_b - z)^2}},$$

wobei $R_a$ und $R_b$ die Krümmungsradien der Cornea-Oberfläche in Richtung der Hauptachsen der Ellipse bezeichnen und z den Abstand des Bearbeitungspunktes (der Höhenlinie) vom Hornhautscheitel. Da z in dem gewählten Zylinderkoordinatensystem (z, Abstand von Hornhautscheitel; r, Abstand zur optischen Achse; φ) dann eine Funktion des radialen Parameters v des Bearbeitungsfeldes ist, ist es zweckmäßig, die radiale Abhängigkeit der Elliptizität durch e(z) = e(z(r)) zu beschreiben.

Die oben genannte Gleichung gilt primär für das unkontaktierte Auge. Bei Anpressen an ein Kontaktglas kommt es in der Regel zu einer Deformation, die bei der Berechnung berücksichtigt werden muss. Dabei spielen der äußere Krümmungsradius der Cornea $R_{Cv}$ und der Krümmungsradius des Kontaktglases $R_G$ eine Rolle. Eine einfache und kompakte Form der Transformation lautet:

$$\varphi' = \varphi$$

$$\alpha' \cdot R' = \alpha \cdot R$$

$$R_G - R' = R_{Cv} - R$$

Weitere Ergänzungen, die in den Gleichungen zu Korrekturtermen führen, sind natürlich möglich und teilweise auch sinnvoll. Obiger Ansatz wird dadurch jedoch nur modifiziert, gilt also im Prinzip weiter. Die genannten Zusammenhänge ermöglichen die Berechnung der Bahnkurven, was auch die Berechnung der Elliptizität mit einschließt. Ein besonders wichtiger Schritt bei den Algorithmen zur Berechnung ist die Vorwärts- und Rückwärtstransformation zwischen natürlichem Augensystem und Kontaktglassystem.

Die Elliptizität der Bahnkurven beträgt für ein Kontaktglas mit einem Krümmungsradius, der etwa dem des menschlichen Auges entspricht, in der Regel weniger als 1.2 (große Hauptachse 10% länger als die kleine Hauptachse). Die Elliptizität beträgt bei einer sphäro-zylindrischen Korrektur mit -2dpt und 1dpt beispielsweise nur etwa 1.03 im zentralen Feldbereich nahe der optischen Achse und wächst mit dem Abstand von der optischen Achse bis zur äußeren Bahnkurve um ca. 10%. Für einen Ansatz spielt die Veränderlichkeit der Elliptizität oder einer entsprechenden Modifikation einer idealen Kreisbahn bei Fehlsichtigkeitskorrektur höherer Ordnung keine störende Rolle, kann also in einer ersten Näherung als konstant angenommen werden.

Es sei noch einmal betont, dass die erfindungsgemäße Verwendung von Höhenlinien sowohl für Ansätze mit wie auch ohne (ebenem oder gekrümmtem) Kontaktglas anwendbar sind, allerdings entfällt bei der Verwendung eines Kontaktglases jeglicher Nachführungsbedarf und es besteht keine Unsicherheit hinsichtlich der vorliegenden Oberflächentopographie.

Wird kein Kontaktglas verwendet, lässt sich die Oberflächentopographie mit geeigneten Verfahren und Vorrichtungen bestimmen und wird anschließend, (analog) ebenso wie die durch den Anpressvorgang festgelegte Topographie im Verfahren berücksichtigt.

Ist die Form der Kontaktglasfläche nicht durch eine Sphäre beschreibbar, sondern folgt einer anderen Raumflächenfunktion, beispielsweise einem Paraboloiden, kann analog der oben angegebenen Transformation ein Transformationsgesetz angegeben werden, das aber den gleichen physikalischen Gedanken folgt.

**Patentansprüche**

1. Vorrichtung zum Ausbilden gekrümmter Schnittflächen (9) in einem transparenten Material, insbesondere in der Augenhornhaut (5), mit einer Laserstrahlungsquelle (S), die Laserstrahlung (3) in das Material (5) fokussiert und dort optische Durchbrüche (8) bewirkt, wobei eine Scaneinrichtung (6, 10), die den Fokuspunkt (7) dreidimensional verstellt, und eine Steuereinrichtung (2) vorgesehen sind, die die Scaneinrichtung (6, 10) ansteuert, um die Schnittfläche (9) durch Aneinanderreihen der optischen Durchbrüche (8) im Material (5) zu bilden, und wobei die Scaneinrichtung (6, 10) zur Verstellung des Fokuspunktes (7) in einer ersten Raumrichtung (z) eine verstellbare Optik (6) aufweist, **dadurch gekennzeichnet, dass** die Steuereinrichtung (2) die Scaneinrichtung (6, 10) so ansteuert, dass der Fokuspunkt (7) in den übrigen zwei Raumrichtungen (x, y) auf einer Bahn geführt ist, der Höhenlinien (17) der Schnittfläche (9) zugrunde liegen, wobei die Höhenlinien in Ebenen liegen, welche im Wesentlichen senkrecht zur ersten Raumrichtung (z) sind, und wobei die Steuereinrichtung (2) die Scaneinrichtung (6, 10) so ansteuert, dass benachbarte Höhenlinien (17.1-17.4) durch einen Übergang (18.1-18.3) miteinander verbunden sind, so dass insgesamt der Fokuspunkt (7) auf einer zusammenhängenden Bahnkurve bewegt ist und die optischen Durchbrüche (8) auf der zusammenhängenden Bahnkurve liegen.

**2.** Vorrichtung nach Anspruch 1, **dadurch gekennzeichnet, dass** die verstellbare Optik eine Teleskopanordnung (6) aufweist.

**3.** Vorrichtung nach einem der obigen Ansprüche, **dadurch gekennzeichnet, dass** die Scaneinrichtung (6, 10) zwei Kippspiegel (11, 12) mit gekreuzten Drehachsen aufweist, um die Verstellung in den übrigen zwei Raumrichtungen (x, y) zu bewirken.

**4.** Vorrichtung nach einem der obigen Ansprüche, **dadurch gekennzeichnet, dass** die Höhenlinien elliptisch mit einer Elliptizität zwischen 1,0 und 1,2 sind.

**5.** Vorrichtung nach einem der obigen Ansprüche, **dadurch gekennzeichnet, dass** die Steuereinrichtung (2) die Abstände der Höhenlinien (17) in der ersten Raumrichtung so wählt, dass die mittleren Abstände zwischen benachbarten Höhenlinien (17) im Wesentlichen, insbesondere innerhalb $\pm$ 10%, konstant sind.

**6.** Vorrichtung nach einem der obigen Ansprüche, **dadurch gekennzeichnet, dass** die Steuereinrichtung (2) den Fokuspunkt (7) jeweils bis auf ein Reststück vollständig entlang der Höhenlinien (17) bewegt und im Reststück durch Verstellung des Fokuspunktes (7) in der ersten Raumrichtung (z) einen Übergang (18) zur nächsten Höhenlinie (17) vornimmt.

**7.** Vorrichtung nach einem der obigen Ansprüche, dadurch gegenzeichnet, dass die Steuereinrichtung (2) bei höheren Krümmungsordnungen der Schnittfläche (9) die Höhenlinien (17) durch Schneiden einer um höhere Krümmungsordnungen bereinigten gekrümmten Schnittflächen (9) mit Ebenen senkrecht zur ersten Raumrichtung (z) bestimmt.

**8.** Vorrichtung nach Anspruch 6, dadurch gegenzeichnet, dass die Steuereinrichtung (2) die Verstellung in der ersten Raumrichtung (z) gemäß dem Einfluss der höheren Krümmungsordnungen verändert, während sie den Fokuspunkt (7) in den übrigen zwei Raumrichtungen (x, y) gemäß der Höhenlinien (17) verstellt, die der bereinigten Schnittfläche (9) ohne höhere Krümmungsordnungen zugeordnet sind.

**9.** Vorrichtung nach einem der obigen Ansprüche, **dadurch gekennzeichnet, dass** mittels eines Kontaktglases (21) der Oberfläche des Materials (5) eine bestimmte Form verliehen ist und dass die Steuereinrichtung (2) die bestimmte Form bei den Höhenlinien berücksichtigt.

**10.** Vorrichtung nach einem der obigen Ansprüche, **dadurch gekennzeichnet, dass** die Steuereinrichtung (2) die Laserstrahlung (3) bezüglich der Erzeugung optischer Durchbrüche (8) abschaltet, solange die Höhenlinie (17) außerhalb eines Sollbereiches des Materials (5) verläuft, in dem die Schnittfläche (9) erzeugt werden soll, wobei insbesondere der Sollbereich (19) entlang der ersten Raumrichtung (z) gesehen kreisförmig ist.

**11.** Vorrichtung nach einem der obigen Ansprüche, **gekennzeichnet durch** eine Einrichtung zum kurzzeitigen Abschalten oder Schwächen des Laserstrahls (3).

**12.** Vorrichtung nach einem der obigen Ansprüche, **dadurch gekennzeichnet, dass** die Übergänge zwischen benachbarten Höhenlinien (17.1-17.4) gleitende Übergänge (18.1-18.4) sind.

**13.** Vorrichtung nach einem der obigen Ansprüche, **dadurch gekennzeichnet, dass** die Steuereinrichtung (2) einen gleichsinnig rotierenden Umlauf des Fokuspunkt bewirkt,

**14.** Vorrichtung nach einem der obigen Ansprüche, **dadurch gekennzeichnet, dass** die Übergänge (18.1-18.3) zwischen benachbarten Höhenlinien (17.1-17.3) entlang Falllinien der Schnittfläche (9) verlaufen.

**15.** Vorrichtung nach einem der obigen Ansprüche, **dadurch gekennzeichnet, dass** die Höhenlinien (17.1-17.3) konzentrisch und als Ellipsen ausgebildet sind.

**Claims**

**1.** Apparatus for forming curved cut surfaces (9) in a transparent material, in particular in the eye cornea (5), having a laser radiation source (S) that focuses laser radiation (3) into the material (5) and causes optical penetrations (8) there, wherein a scanning device (6, 10), which three-dimensionally adjusts the focus point (7), and a control device (2), which controls the scanning device (6, 10) to form the cut surface (9) by arranging the optical penetrations (8) next to one another in the material (5), are provided and wherein the scanning device (6, 10) comprises an adjustable optical unit (6) for adjusting the focus point (7) in a first spatial direction (z), **characterized in that** the control device (2) controls the scanning device (6, 10) in a manner such that the focus point (7) is guided in the remaining two spatial directions (x, y) on a path based on contour lines (17) of the cut surface (9), wherein the contour lines lie in planes that are substantially perpendicular to the first spatial direction (z), and wherein the control device (2) controls the scanning device (6, 10) in a manner such that adjacent contour lines (17.1-17.4) are connected by a transition (18.1-18.3) so that, overall, the focus point (7) is moved along a contiguous trajectory and the optical penetrations (8) lie on the contiguous trajectory.

2. Apparatus according to Claim 1, **characterized in that** the adjustable optical unit comprises a telescope arrangement (6).

3. Apparatus according to either of the above claims, **characterized in that** the scanning device (6, 10) comprises two tilt mirrors (11, 12) with intersecting rotational axes for causing the adjustment in the remaining two spatial directions (x, y).

4. Apparatus according to one of the above claims, **characterized in that** the contour lines are elliptical with an ellipticity of between 1.0 and 1.2.

5. Apparatus according to one of the above claims, **characterized in that** the control device (2) chooses the distances of the contour lines (17) in the first spatial direction such that the average distances between adjacent contour lines (17) are substantially, in particular within ±10%, constant.

6. Apparatus according to one of the above claims, **characterized in that** the control device (2) moves the focus point (7) in each case completely, save for a residual piece, along the contour lines (17) and in the residual piece implements a transition (18) to the next contour line (17) by adjusting the focus point (7) in the first spatial direction (z).

7. Apparatus according to one of the above claims, **characterized in that** the control device (2) at higher orders of curvature of the cut surface (9) determines the contour lines (17) by cutting a curved cut surface (9), corrected by higher orders of curvature, with planes perpendicular to the first spatial direction (z).

8. Apparatus according to Claim 6, **characterized in that** the control device (2) changes the adjustment in the first spatial direction (z) in accordance with the influence of the higher orders of curvature while it adjusts the focus point (7) in the remaining two spatial directions (x, y) in accordance with the contour lines (17) assigned to the corrected cut surface (9) without higher orders of curvature.

9. Apparatus according to one of the above claims, **characterized in that** the surface of the material (5) is given a specific shape by means of a contact lens (21) and **in that** the control device (2) takes the specific shape into account for the contour lines.

10. Apparatus according to one of the above claims, **characterized in that** the control device (2) switches off the laser radiation (3) with respect to the production of optical penetrations (8) as long as the contour line (17) extends outside a target region of the material (5) in which the cut surface (9) is to be produced, wherein in particular the target region (19), viewed along the first spatial direction (z), is circular.

11. Apparatus according to one of the above claims, **characterized by** a device for briefly switching off or diminishing the laser beam (3).

12. Apparatus according to one of the above claims, **characterized in that** the transitions between adjacent contour lines (17.1-17.4) are smooth transitions (18.1-18.4) .

13. Apparatus according to one of the above claims, **characterized in that** the control device (2) causes a circulatory path of the focus point in the same direction.

14. Apparatus according to one of the above claims, **characterized in that** the transitions (18.1-18.3) between adjacent contour lines (17.1-17.3) extend along fall lines of the cut surface (9).

**15.** Apparatus according to one of the above claims, **characterized in that** the contour lines (17.1-17.3) are concentric and embodied in the form of ellipses.

## Revendications

**1.** Dispositif pour la formation de surfaces de coupe courbées (9) dans un matériau transparent, notamment dans la cornée de l'œil (5), pourvu d'une source de rayonnement laser (S), qui focalise un rayonnement laser (3) dans le matériau (5) et y réalise des percées optiques (8), un dispositif de balayage (6, 10), qui règle tridimensionnellement le point de focalisation (7), et un dispositif de commande (2) étant prévus, qui commande le dispositif de balayage (6, 10), afin de former la surface de coupe (9) par juxtaposition des percées optiques (8) dans le matériau (5), et le dispositif de balayage (6, 10) comprenant un élément optique réglable (6) pour le réglage du point de focalisation (7) dans une première direction de l'espace (z), **caractérisé en ce que** le dispositif de commande (2) commande le dispositif de balayage (6, 10) de telle sorte que le point de focalisation (7) soit guidé dans les deux autres directions de l'espace (x, y) sur une trajectoire, dont les courbes de niveau (17) sont à la base de la surface de coupe (9), les courbes de niveau étant situées dans des plans, qui sont essentiellement perpendiculaires à la première direction de l'espace (z), et le dispositif de commande (2) commandant le dispositif de balayage (6, 10) de telle sorte que des courbes de niveau voisines (17.1-17.4) soient reliées l'une avec l'autre par une transition (18.1-18.3), de telle sorte qu'au total, le point de focalisation (7) soit déplacé sur une trajectoire courbe continue et que les percées optiques (8) soient situées sur la trajectoire courbe continue.

**2.** Dispositif selon la revendication 1, **caractérisé en ce que** l'élément optique réglable comprend un agencement télescopique (6).

**3.** Dispositif selon l'une quelconque des revendications précédentes, **caractérisé en ce que** le dispositif de balayage (6, 10) comprend deux miroirs inclinables (11, 12) pourvus d'axes de rotation croisés, afin de réaliser le réglage dans les deux autres directions de l'espace (x, y).

**4.** Dispositif selon l'une quelconque des revendications précédentes, **caractérisé en ce que** les courbes de niveau sont elliptiques avec une ellipticité comprise entre 1,0 et 1,2.

**5.** Dispositif selon l'une quelconque des revendications précédentes, **caractérisé en ce que** le dispositif de commande (2) choisi les écarts des courbes de niveau (17) dans la première direction de l'espace de telle sorte que les écarts moyens entre des courbes de niveau voisines (17) soient essentiellement constants, notamment dans les limites de $\pm$ 10 %.

**6.** Dispositif selon l'une quelconque des revendications précédentes, **caractérisé en ce que** le dispositif de commande (2) déplace le point de focalisation (7) entièrement le long des courbes de niveau (17), respectivement à l'exception d'une partie restante, et effectue dans la partie restante une transition (18) jusqu'à la courbe de niveau suivante (17) par réglage du point de focalisation (7) dans la première direction de l'espace (z).

**7.** Dispositif selon l'une quelconque des revendications précédentes, **caractérisé en ce qu'**en cas d'ordres de courbure plus élevés de la surface de coupe (9), le dispositif de commande (2) détermine les courbes de niveau (17) par découpe d'une surface de coupe courbée (9) corrigée pour des ordres de courbure plus élevés avec des plans perpendiculaires à la première direction de l'espace (z).

**8.** Dispositif selon la revendication 6, **caractérisé en ce que** le dispositif de commande (2) modifie le réglage dans la première direction de l'espace (z) selon l'effet des ordres de courbure plus élevés, tandis qu'il règle le point de focalisation (7) dans les deux autres directions de l'espace (x, y) selon les courbes de niveau (17), qui sont associées à la surface de coupe corrigée (9) sans ordres de courbre plus élevés.

**9.** Dispositif selon l'une quelconque des revendications précédentes, **caractérisé en ce qu'**une forme déterminée est conférée à la surface du matériau (5) au moyen d'un verre de contact (21) et **en ce que** le dispositif de commande (2) prend en compte la forme déterminée pour les courbes de niveau.

**10.** Dispositif selon l'une quelconque des revendications précédentes, **caractérisé en ce que** le dispositif de commande (2) arrête le rayonnement laser (3) au regard de la génération de percées optiques (8) tant que la courbe de niveau (17) passe à l'extérieur d'une zone de consigne du matériau (5), dans laquelle la surface de coupe (9) doit être générée, la zone de consigne (19) étant notamment circulaire telle que vue le long de la première direction de l'espace (z).

11. Dispositif selon l'une quelconque des revendications précédentes, **caractérisé par** un dispositif pour l'arrêt ou l'affaiblissement bref du rayon laser (3).

12. Dispositif selon l'une quelconque des revendications précédentes, **caractérisé en ce que** les transitions entre des courbes de niveau voisines (17.1-17.4) sont des transitions glissantes (18.1-18.4).

13. Dispositif selon l'une quelconque des revendications précédentes, **caractérisé en ce que** le dispositif de commande (2) réalise une circulation rotative de même sens du point de focalisation.

14. Dispositif selon l'une quelconque des revendications précédentes, **caractérisé en ce que** les transitions (18.1-18.3) entre des courbes de niveau voisines (17.1-17.3) passent le long de lignes de pente de la surface de coupe (9).

15. Dispositif selon l'une quelconque des revendications précédentes, **caractérisé en ce que** les courbes de niveau (17.1-17.3) sont configurées concentriquement et sous la forme d'ellipses.

Fig. 1

FIG.2

FIG.3

FIG.4

FIG.8a

FIG.8b

FIG.5

FIG.6

FIG.7

FIG.9

FIG.10

FIG.11

FIG.12

FIG.13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5984916 A
- US 6110166 A